# EUROPEAN PATENT APPLICATION

(11) **EP 3 871 687 A1**
(43) Date of publication of application: **01.09.2021**
(21) Application number: 20159824.0
(22) Date of filing: 27.02.2020
(51) Int. Cl.: A61K 38/47, A61P 3/00

(54) **ENZYME REPLACEMENT THERAPY FOR TREATING POMPE DISEASE**

(71) Applicant: eleva GmbH, 79108 Freiburg (DE)
(72) Inventor: DABROWSKA-SCHLEPP, Paulina, 79211 Denzlingen (DE); KRIEGHOFF, Nicola, 79108 Freiburg (DE); SCHAAF, Andreas, 79104 Freiburg (DE); BERG, Birgit, 79117 Freiburg (DE); BUSCH, Andreas, 79285 Ebringen (DE)
(74) Representative: SONN Patentanwälte OG

(57) **Abstract**

The present invention relates to a method of treating a patient with Pompe disease comprising providing a composition of a recombinant acid alpha-glucosidase, which comprises a glycosylation structure of formula 1, wherein a "G" represents N-acetylglucosamine, a "M" represents mannose, and a "G/M" represents a terminal N-acetylglucosamine or terminal mannose, wherein one but not both of the "G/M" subunits may be absent in which case the present "G/M" subunit may represent two terminal mannose subunits, wherein one or more of the "G" or "M" subunits are not α1,3-fucosylated, not α1,6-fucosylated and not β1,2-xylosylated, and wherein any one of "G", "M" or "G/M" may be methylated; and said glycosylation structure of formula 1 is present in at least 20% of all glycosylation structures of the recombinant acid alpha-glucosidases of the composition; and administering the composition to the patient.

## Description

The present invention relates to the field of modified glycosylation patterns on therapeutic proteins for enzyme replacement therapy.

### Background of the invention

WO 2016/146760 A1 describes recombinant proteins with paucimannosidic glycosylations on lysosomal proteins to treat lysosomal storage diseases. A paucimannosidic glycosylation comprises N-acetylglucosamine (GlcNAc) and mannose (Man) residues and has the structure -GlcNAc-GlcNAc-Man<(Man)₂, with "Man<(Man)₂" being two branching mannose residues bound to a connecting mannose. These paucimannosidic glycosylation structures are found as N-glycans, i.e. bound to an Asn residue of the proteins. These structures are also called simply "Man3" or "MM", which characterizes the two terminal mannose residues. Uptake of these proteins was found to be among others via the mannose receptor.

Pompe disease (also Glycogen storage disease type II; OMIM #232300) is an autosomal recessive lysosomal storage disorder (LSD) caused by deficiency of lysosomal acid alpha-1,4-glucosidase (or just acid alpha-glucosidase, abbreviated "GAA"). Mutations in GAA gene result in GAA enzyme deficiency (Martiniuk et al., Am J Hum Genet, 1990, 47(3): p. 440-5; Lam et al., Neurology, 2003, 60(4): p. 715-7), which is a lysosomal enzyme catalyzing the degradation of glycogen. The result of the GAA deficiency is an ubiquitous lysosomal and non-lysosomal accumulation of glycogen. The most heavily affected tissues are heart, skeletal muscle, liver, and the nervous system. There are basically two forms of this disease: an infantile form, which presents before the age of 12 months and a late onset form, characterized by an onset at the age of 12 months to adulthood. While cardiomegaly due to heart muscle insufficiency as inadequate growth compensation is particularly apparent in the infantile form, skeletal muscle insufficiency is prevalent in both forms but more prominent in the late onset form. Further indications are hypotonia, cardiomyopathy, respiratory distress and muscle weakness.

Since 2006 a licensed enzyme replacement therapy (ERT)is available for all Pompe patients: Alglucosidase alfa (Myozyme®/Lumizyme®), a recombinant human GAA (rhGAA) produced in Chinese hamster ovary cells carrying mannose-6-phosphate glycans, is taken up into cells via the cation independent mannose 6-phosphate (M6P) receptor (Van Hove et al., PNAS, 1996, 93(1): p. 65-70). It gets delivered directly to the lysosomes by receptor mediated endocytosis. Treatment with alglucosidase alfa leads to amelioration of disease symptoms in cardiac muscle in most cases, but its therapeutic effect in skeletal muscle and other tissues is limited. Results of long-term clinical studies show stabilization and/or improvement in walking distance and respiratory function, while weakness of axial skeletal muscles usually persists or further declines (Schoser et al., Journal of neurology, 2017, 264(4): p. 621-630). Due to the restricted clinical efficacy of this ERT, there is an ongoing need for improvements of the administrated enzyme in terms of stability and delivery efficacy.

Several attempts have been made to improve the GAA enzyme production in terms of stability while reducing production costs using different cell systems such as rice calli (Jung et al., Journal of biotechnology, 2016, 226: 44-53), tabacco (Martiniuk et al., Applied biochemistry and biotechnology, 2013, 171(4): 916-26), and rabbit milk (Bijvoet et al., Human molecular genetics, 1999, 8(12): 2145-53). While GAA produced in rabbit milk showed no clinical efficacy in a PhI/II clinical trial, no further clinical data exists regarding the production in tobacco and rice calli.

Other attempts aimed at improving enzyme uptake and enzyme efficacy in order to alleviate disease symptoms. These attempts include different modifications of alglucosidase alfa, e.g. crafting of M6P analogues (El Cheikh et al. Angewandte Chemie, 2016. 55(47): 14774-14777), increasing the number of M6P residues on the enzyme (Zhu et al., Molecular therapy: the journal of the American Society of Gene Therapy, 2009, 17(6): 954-63), or coadministration with a small molecule pharmacological chaperone (LeBowitz et al. PNAS USA, 2004, 101(9): 3083-8, Xu et al. JCI Insight, 2019, 4(5), e125358).

Despite these efforts, there is an ongoing need to improve the efficacy of therapies for the treatment of Pompe disease.

### Summary of the invention

The present invention provides composition suitable for treating a patient with Pompe disease. The composition comprises a recombinant acid alpha-glucosidase that comprises a glycosylation structure of formula 1: 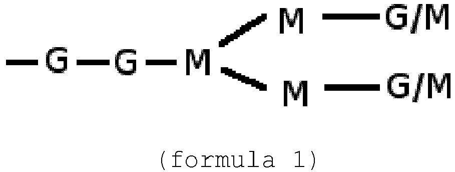 wherein a "G" represents N-acetylglucosamine, a "M" represents mannose, and a "G/M" represents a terminal N-acetylglucosamine or terminal mannose or two terminal mannose subunits or may be absent, wherein one or more of the "G" or "M" subunits may be α1,3-fucosylated, α1,6-fucosylated and/or β1,2-xylosylated, and wherein any one of "G", "M" or "G/M" may be methylated; and said glycosylation structure of formula 1 is present in at least 20% of all the recombinant acid alpha-glucosidase's glycosylation structures in the composition.

The invention further provides the composition for use in the treatment of a patient suffering from Pompe disease.

The invention further provides a method of treating Pompe disease in a patient comprising administering the acid alpha-glucosidase composition of the invention to the patient.

All aspects of the invention are interrelated and a disclosure of a composition also relates to the methods of treatment, e.g. as a disclosure of a composition that is to be used in the treatment and vice-versa, e.g. a disclosure of a method of treatment also reads on the composition that is suitable for such a treatment.

### Detailed description of the invention

The present invention is based on the new discovery that acid alpha-glucosidase with new glycosylation structures is suitable to treat Pompe disease. In particular, it was found that the modified proteins with these structures have better uptake into differentiated muscle cells, including skeletal muscle cells and heart muscle cells. Muscle cells are an important target since current enzyme replacement therapy using normal human acid alpha-glucosidase (rhGAA, alglucosidase alfa), falls short in targeting these cells, which leads to an insufficient treatment of progressive muscle weakness in Pompe disease patients. This shortcoming of existing treatment options is particularly prevalent in case of skeletal muscle cell loss.

The present invention provides a composition of acid alpha-glucosidase in the treatment of Pompe disease that exhibits surprisingly good uptake and activity properties in differentiated muscle cells. This result is surprising since the commercially available ERT relies on M6P-mediated endocytosis, while the composition provided in the present invention has terminal mannose residues or terminal N-acetylglucosamine residues, thereby showing that M6P-residues are not necessary for cellular uptake in ERT.

The invention provides a composition of a recombinant acid alpha-glucosidase ("GAA"), which comprises a glycosylation structure of formula 1 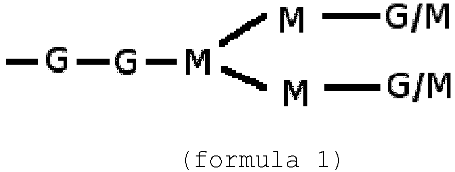 wherein a "G" represents N-acetylglucosamine, a "M" represents mannose, and a "G/M" represents a terminal N-acetylglucosamine (also "GlcNAc") or terminal mannose (also "Man"), wherein one but not both of the "G/M" subunits may represent two terminal mannose subunits, and one G/M may be absent, wherein one or more of the "G" or "M" subunits are not α1,3-fucosylated, not α1,6-fucosylated and not β1,2-xylosylated, and wherein any one of "G", "M" or "G/M" may be methylated; and said glycosylation structure of formula 1 is present in at least 20% of all glycosylation structures of the recombinant acid alpha-glucosidases of the composition.

All % amounts are molar ratios unless explicitly stated otherwise.

The invention in particular provides a method of treating a patient with Pompe disease comprising providing a composition of the recombinant acid alpha-glucosidase, which comprises a glycosylation structure of formula 1 and administering the composition to the patient. The composition is usually administered in pharmaceutically active amounts to treat Pompe disease.

Several GAA compositions with different glycostructures have been tested and compared to alglucosidase alfa and it is apparent that the inventive glycostructures provide an alternative mediator for cellular and lysosomal uptake of the GAA. Surprisingly, in relevant cells, such as differentiated muscle cells, the inventive GAA surpasses uptake of alglucosidase alfa due to the different glycosylation structures. The superior uptake suggests an improved efficacy.

In particular it appears that lysosomal glycogen accumulation in cells of Pompe patients is not the only target. Further glycogen accumulation also occurs in the cytoplasm. Without being bound to a particular theory, it appears that the inventive GAA compositions are capable to target and degrade both glycogen deposits, in the lysosome and in the cytoplasm. This is a particular advantage when targeting skeletal muscle cells, especially in the treatment of late onset Pompe disease, which is particular preferred treatment indication for the inventive composition.

Among others, possible glycosylation structure of the GAA of the present invention are selected from formulas 2-5: 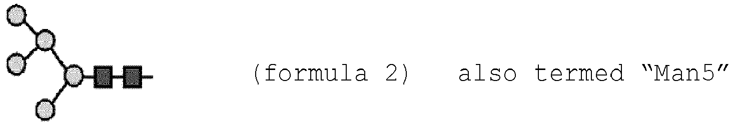 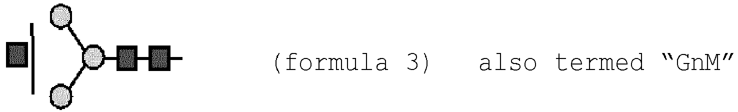 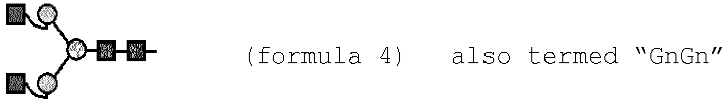 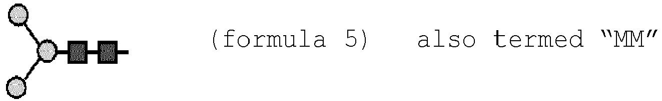 wherein a circle represents mannose and a square represents N-acetylglucosamine, wherein any mannose or N-acetylglucosamine may be methylated. A square with a bar means that the N-acetylglucosamine can be bound to any one of the mannose residues adjacent to the bar.

In preferred embodiments of the invention, the glycosylation structure of formula 1 is present in at least 30%, especially preferred at least 40%, at least 50%, at least 60%, at least 70%, at least 80%, at least 90%, of all glycosylation structures of the recombinant acid alpha-glucosidases of the composition.

The reference to all glycosylation structures of the recombinant acid alpha-glucosidases means the percentage is found in the pattern of a glycosylation structure of the GAA of the composition. One GAA protein may be glycosylated up to 7 times due to the presence of 7 N-glycosylation sites. One GAA protein may be heterogeneously glycosylated by different glycosylation structures or homogenously glycosylated by one type of glycosylation structure. In the above values for the composition, the overall values are determined.

Preferably, in formula 1, none or one of the "G/M" subunits may be absent. Also preferred, one "G/M" subunit may represent two terminal mannose subunits. In particular in case one "G/M" is absent, this other "G/M" may represent one or two terminal mannose subunits. "Two terminal" mannose subunits means that both mannose residues are terminal as in <(Man)₂, i.e. a branched structure with the second Man being terminal. Also possible for "G/M" is a -Man-Man structure, with one Man being a connecting Man and the other Man being terminal.

In preferred embodiments at least one "G/M" of formula 1 is N-acetylglucosamine, preferably wherein both "G/M" of formula 1 are N-acetylglucosamine.

A terminal residue means that the glycan ends on this residue and no further residues such as Man, GlcNAc, sialyl, galactosyl are connected in a 2-, 3-, 4- or 6- position (i.e. nonreducing end of glycan).

Preferably one or more of the "G" or "M" subunits are not α1,3-fucosylated, and/or not α1,6-fucosylated and/or not β1,2-xylosylated, especially preferred not α1,3-fucosylated and not β1,2-xylosylated. These residues may lead to reduced performance of the inventive GAA *in vivo* and should be avoided. α1,3-fucosylations and α1,6-fucosylatations are found commonly on the reducing GlcNAc of the glycosylation structure. A β1,2-xylosylation is usually found at the non-terminal mannose connecting to the starting (reducing) -GlcNAc-GlcNAc structure. According to the invention, preferably a α1,3-fucosylation and/or β1,2-xylosylation is prevented or reduced because these modifications may inhibit the GAA. These may be present when using a plant expression system but can be avoided by supressing the plant α1,3-fucosyltransferase and/or β1,2-xylosyltransferase (WO00/49153 A1, WO01/64901 A1, WO2004/057002 A2). α1,6-fucosylation may or may not be present. It is uncommon in plants but may be achieved by introduction of a α1,6-fucosyltransferase into an expressing plant cell. Preferably, the composition comprises at most 20% of recombinant acid alpha-glucosidase with a glycosylation structure of formula 1 that is at least either α1,3-fucosylated, α1,6-fucosylated or β1,2-xylosylated or a combination thereof.

In preferred embodiments α1,3-fucose and/or β1,2-xylose each may be present in 0% to 30%, especially preferred 0.25% to 20% or 0.5% to 10%, more preferred less than 5% or less than 1 %, of all glycosylation structures of the recombinant acid alpha-glucosidases of the composition.

In preferred embodiments α1,6-fucose may be present in 0% to 90%, especially preferred 5% to 70% or 10% to 50%, more preferred less than 35% or less than 20%, of all glycosylation structures of the recombinant acid alpha-glucosidases of the composition.

Any one of "G", "M" or "G/M" may be methylated, especially O-methylated. This is a common modification that does not impair activity and can or may not be present. Especially preferred, the glycosylation structure comprises none, one or two methylated residues selected from "G", "M" or "G/M". Especially preferred, a methylation may be present in 0% to 30%, especially preferred 0.5% to 20% or 1% to 10%, of all glycosylation structures of the recombinant acid alpha-glucosidases of the composition.

In preferments the glycosylation structure of formula 1 is glycosylated in at least 10% of the recombinant acid alpha-glucosidase's N-glycosylation sites for the recombinant acid alpha-glucosidases of the composition. The usual glycosylation sites of GAA are N140, N233, N390, N470, N652, N882 and N925 (amino acid annotation of human GAA, (see Roig-Zamboni et al., Nature Communications, 2017, 8:1111; sequence database UniProtKB, entry "P10253", sequence version of 11 Jan 2011)). Preferably N140, N233, N390, N470, N652 are selected for glycosylation; N882 and N925 are rarely glycosylated. This figure refers to all combined glycosylation sites of all GAA in the composition. In preferred embodiments at least 20%, especially preferred at least 30%, at least 40%, at least 50%, of the recombinant acid alpha-glucosidase's N-glycosylation sites of the acid alpha-glucosidases of the composition are glycosylated.

In preferred embodiments, 1, 2, 3, 4, 5, 6 or 7 glycosylation sites are glycosylated in a GAA of the inventive composition. Preferably at least 40%, especially preferred at least 60% or at least 80% of the GAA of the composition are glycosylated in at least 1 glycosylation site. Preferably at least 35%, especially preferred at least 50% or at least 70% of the GAA of the composition are glycosylated in at least 2 glycosylation sites. Preferably at least 30%, especially preferred at least 45% or at least 60% of the GAA of the composition are glycosylated in at least 3 glycosylation sites. Preferably at least 25%, especially preferred at least 40% or at least 50% of the GAA of the composition are glycosylated in at least 4 glycosylation sites. Preferably the composition comprises less than 50%, preferably less than 25%, not-glycosylated GAA.

In preferred embodiments, especially for the inventive treatment uses, the composition comprises at least 30% of recombinant acid alpha-glucosidase with a glycosylation structure of formula 1, wherein one or both, preferably both, G/M are terminal N-acetylglucosamine (GlcNAc). These glycosylation structures with a GlcNAc structure are particularly effective. Especially preferred are even higher amounts thereof, e.g. wherein the GAA of the inventive composition has at least 40% of its glycosylation structures according to formula 1 with one or both, preferably both, "G/M" as terminal N-acetylglucosamine.

In preferred embodiments, especially for the inventive treatments, the glycosylation structure is preferably further defined as a glycosylation structure selected from formulas 2-4: 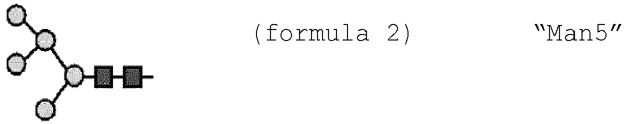 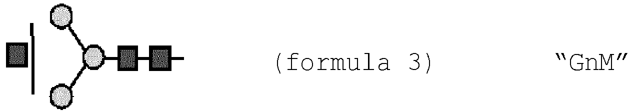 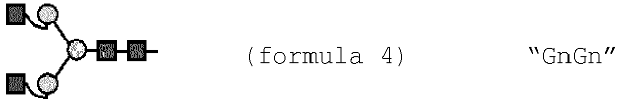 wherein a circle represents mannose and a square represents N-acetylglucosamine, wherein any mannose or N-acetylglucosamine may be methylated. This selection is preferably present in at least 40%, especially preferred at least 50%, at least 60%, at least 70%, at least 80%, at least 90%, of the (N-glycan) glycosylation structures of the GAA of the composition.

Especially preferred, the combined amount of the glycosylation structure of formula 3 and formula 4, which may be methylated, is at least 70%, more preferred at least 80%, or at least 90%, of all glycosylation structures of the recombinant acid alpha-glucosidases of the composition.

In particular preferred, the glycosylation structure of formula 4 is present in at least 60%, more preferred at least at least 70%, at least 80%, at least 90%, of all glycosylation structures of the recombinant acid alpha-glucosidases of the composition.

The GAA may be fragmented in the lysosome (Moreland et al., JBC 280(8), 6780-6791, 2005). Preferably the GAA may be fragmented to a GAA main fragment corresponding to amino acids (aa) 122-782 of human GAA according to sequence database UniProtKB, entry "P10253", sequence version of 11 Jan 2011. Preferably, the GAA main fragment is in complex with one or more of fragments corresponding to amino acids 78-113 or corresponding to amino acids 792-952 of human GAA according to sequence database UniProtKB, entry "P10253", sequence version of 11 Jan 2011. In some embodiments, the main fragment is further fragmented to a fragment corresponding to amino acids 122-199 of human GAA according to sequence database UniProtKB, entry "P10253", sequence version of 11 Jan 2011 is in complex, and a fragment corresponding to amino acids 204-782 of human GAA according to sequence database UniProtKB, entry "P10253", sequence version of 11 Jan 2011. These may also be in complex with the aa78-113 and aa792-952 fragments. The GAA of the inventive composition may be suitable for such fragmentation in some embodiments.

The invention also provides the GAA composition as a medicament. The present invention provides the inventive composition for use in the treatment of a patient suffering from Pompe disease or any patient suffering from a glycogen storage disease, or any disease suffering from a GAA insufficiency.

Also provided is the manufacture of a pharmaceutical composition for the treatment of Pompe disease or any patient suffering from a glycogen storage disease, or any disease suffering from a GAA insufficiency comprising the inventive GAA composition. The patient can be a mammalian, especially human or a non-human animal, such as a rodent, a dog, cat, horse, cow, camel, pig. Preferably the GAA is from the same species as the patient in order to prevent immunoreactions against the protein's amino acid chain.

The terms, "treat" or "treatment", as used herein, refers to amelioration of one or more symptoms associated with the disease, prevention or delay of the onset of one or more symptoms of the disease, and/or lessening of the severity or frequency of one or more symptoms of the disease. For example, treatment can refer to improvement of cardiac status (e.g., increase of end-diastolic and/or end-systolic volumes, or reduction, amelioration or prevention of the progressive cardiomyopathy that is typically found in Pompe disease) or of pulmonary function (e.g., increase in crying vital capacity over baseline capacity, and/or normalization of oxygen desaturation during crying); improvement in neurodevelopment and/or motor skills (e.g., increase in AIMS score); reduction of glycogen levels in tissue of the individual affected by the disease; or any combination of these effects. In one preferred embodiment, treatment includes improvement of glycogen clearance, particularly in reduction or prevention of Pompe disease-associated cardiomyopathy. The terms, "improve", "increase" or "reduce", as used herein, indicate values that are relative to a baseline measurement, such as a measurement in the same individual prior to initiation of the treatment described herein, or a measurement in a control individual (or multiple control individuals) in the absence of the treatment described herein. A "control individual" is an individual afflicted with the same form of Pompe disease (either infantile or late (juvenile or adult) onset) as the individual being treated, who is about the same age as the individual being treated (to ensure that the stages of the disease in the treated individual and the control individual(s) are comparable).

In some embodiments the patient suffers from glycogen deposits in muscle cells, e.g. heart and/or skeletal muscle cells. The patient may suffer from muscle loss, in particular skeletal muscle loss and/or heart muscle loss. The patient may suffer from infantile or late (juvenile or adult) onset Pompe disease.

In some embodiments, the invention provides a pharmaceutical composition comprising a recombinant acid alpha-glucosidase, which comprises a glycosylation structure of formula 1 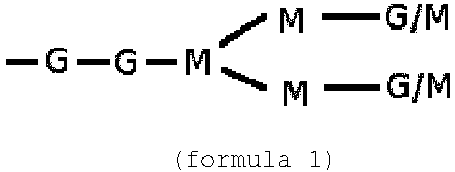 wherein a "G" represents N-acetylglucosamine, a "M" represents mannose, and any one of the "G/M" represents one or two terminal mannose or a terminal N-acetylglucosamine, wherein the other "G/M" is absent or represents a terminal mannose, wherein one or more of the "G" or "M" subunits are not α1,3-fucosylated, not α1,6-fucosylated and not β1,2-xylosylated, and wherein any one of "G", "M" or "G/M" may be methylated; and said glycosylation structure of formula 1 is present in at least 20% of all glycosylation structures of the recombinant acid alpha-glucosidases of the composition.

In some embodiments the inventive GAA may or preferably may not have a lysosomal targeting peptide such as a GILT-tag (WO2008/063511 A2).

In preferred embodiment of the composition the glycosylation structure is further defined as a glycosylation structure selected from formula 2 or formula 3: 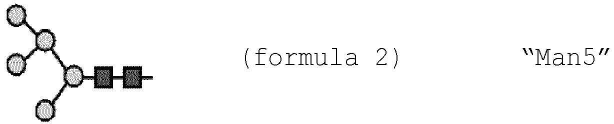 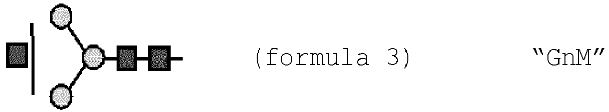 wherein a circle represents mannose and a square represents N-acetylglucosamine, wherein any mannose or N-acetylglucosamine may be methylated, and wherein preferably the glycosylation structure selected from formula 2 is present in at least 60%, preferably at least 70% or at least 80%, of all glycosylation structures of the recombinant acid alpha-glucosidases of the composition.

Further preferred, in all embodiments of the invention, the acid alpha-glucosidase is bound to a biocompatible polymer, preferably wherein the acid alpha-glucosidase is PEGylated. A conjugation with a biocompatible polymer can increase bioavailability and in vivo half-life when administered to a patient. PEGylation is especially preferred to achieve these functions and to increase solubility. Especially preferred is a reversible conjugation, leading to a at least partial loss of the PEGylation in vivo, e.g. by introducing a hydrolysable bond, such as a Schiff base, so as not to interfere with cellular uptake. Also as a measure to reduce cellular uptake interference, the PEGylation can be of short PEG chains, such as PEG with 4 to 1000, preferably 8 to 100 or 10 to 50 subunits. Instead of PEG, any short hydrophilic biocompatible polymer can be used to increase half-life, preferably with a molecular weight of less than 100 kDa, less than 10kDa or less than 1 kDa. Preferably PEG has 2-200, preferably 3 to 100 or 4 to 50, glycol subunits. The lysosomatic protein can be PEGylated via a linking moiety as means for indirect attachment of the PEG molecule. Alternatively, also direct attachment is possible.

The GAA may be not-crosslinked or crosslinked, e.g. to bind monomers to crosslinked dimers or multimers. Crosslinking can be done by the same conjugation as the binding to biopolymers, preferably by PEG binding.

The dose for administration is preferably a dose of 0.05 to 200 mg/kg body weight, preferably of 0.1 to 100 mg/kg body weight, especially preferred of 0.3 to 50 mg/kg body weight, even more preferred 5 to 30 mg/kg body weight such as 5, 10 or 20 mg/kg body weight or any ranges between these values.

Since there is no cure for Pompe disease, a chronic treatment is required with repeated administrations of the enzyme replacement (GAA) in regular intervals. Preferably the composition is administered at an interval of 1 to 30 days, preferably of 2 to 25 days, more preferred of 3 to 23, or even of 4 to 22 days, of 5 to 21 days, of 6 to 20 days, of 7 to 19 days, of 8 to 18 days, of 9 to 17 days, of 10 to 16 days, or of 11 to 15 days. Especially preferred are 14 day intervals. Administration in such intervals allows steady enzyme activity in the cells, countering GAA clearance. The intervals can be extended in case of a conjugation with a biocompatible polymer, especially PEGylation, such as to 28 day intervals or longer.

The GAA composition may be administered by any route that leads to a functional enzyme reaching the vascular system, especially the blood stream. Preferred is intravenous (i.v.) infusion. Further routes of administration include intraperitoneal (i.p.), intramuscular (i.m.) and subcutaneous (s.c.) administration. Non-i.v. routes, in particular i.p., i.m. and s.c., benefit from better patient acceptance and usually the benefit outweighs the reduced target tissue distribution. Furthermore, pharmacokinetic profiles of the non-i.v. administrations are beneficial as the therapeutic enzyme is available in patient's plasma over a prolonged time period. In case of late onset Pompe disease, which markedly affects skeletal muscle a i.m. administration is preferred. The GAA composition may be administered directly to a muscle that is in need of a treatment to reduce glycogen accumulation.

The composition can be formulated in accordance with the routine procedures as a pharmaceutical composition adapted for administration to human beings. For example, in a preferred embodiment, a composition for intravenous administration typically is a solution in sterile isotonic aqueous buffer. Where necessary, the composition may also include a solubilizing agent and a local anesthetic to ease pain at the site of the injection. Generally, the ingredients are supplied either separately or mixed together in unit dosage form, for example, as a dry lyophilized powder or water free concentrate in a hermetically sealed container such as an ampoule, vial or sachet indicating the quantity of active agent. Where the composition is to be administered by infusion, it can be dispensed with an infusion bottle containing sterile pharmaceutical grade water, saline or dextrose/water. Where the composition is administered by injection, an ampule of sterile water for injection or saline can be provided so that the ingredients may be mixed prior to administration.

The GAA composition can be formulated with a physiologically acceptable carrier or excipient to prepare a pharmaceutical composition. The carrier and composition can be sterile. The formulation should suit the mode of administration.

Suitable pharmaceutically acceptable carriers include but are not limited to water, salt solutions (e.g., NaCl), saline, buffered saline, alcohols, glycerol, ethanol, gum arabic, vegetable oils, benzyl alcohols, polyethylene glycols, gelatin, carbohydrates such as lactose, amylose or starch, sugars such as mannitol, sucrose, or others, dextrose, magnesium stearate, talc, silicic acid, viscous paraffin, perfume oil, fatty acid esters, hydroxymethylcellulose, polyvinyl pyrolidone, etc. , as well as combinations thereof. The pharmaceutical preparations can, if desired, be mixed with auxiliary agents (e.g., lubricants, preservatives, stabilizers, wetting agents, emulsifiers, salts for influencing osmotic pressure, buffers, coloring, flavoring and/or aromatic substances and the like) which do not deleteriously react with the active compounds or interference with their activity. In a preferred embodiment, a water-soluble carrier suitable for intravenous administration is used.

The composition, if desired, can also contain minor amounts of wetting or emulsifying agents, or pH buffering agents. The composition can be a liquid solution, suspension, emulsion, tablet, pill, capsule, sustained release formulation, or powder. Binders and carriers can be added. A formulation can include standard carriers such as pharmaceutical grades of mannitol, lactose, starch, magnesium stearate, polyvinyl pyrollidone, sodium saccharine, cellulose, magnesium carbonate, etc.

To increase pharmaceutical targeting diversity, the inventive GAA composition can be combined with other Pompe disease therapies, such as administration of the diversely glycosylated GAA of Alglucosidase alfa. Of course, also the inventive compositions can be combined. Especially preferred is a mixture of GAA compositions with glycosylations according to formula 2, 3 and 4, especially preferred with formula 2 and 4.

The inventive lysosomal protein with paucimannosidic glycosylation can also be combined with a chaperon, in particular a specific or non-specific chaperon of GAA, such as 1-deoxynojirimycin or a derivative thereof according to US2006/0264467 A1.

In preferred embodiments, the GAA is produced in plants, such as a bryophythe or a cell thereof. Preferably the bryophyte plant or cell is a moss, preferably *P. patens,* plant or cell. The bryophyte may be any bryophyte but is preferably selected from moss, liverwort or hornwort, especially preferred of the class *bryopsida* or of the genera *Physcomitrella, Funaria, Sphagnum, Ceratodon, Marchantia* and *Sphaerocarpos. Physcomitrella patens* is a particularly preferred system as it has a high rate of homologous recombination. Accordingly, the invention also provides a method of producing a GAA composition by recombinantly expressing the GAA in such a plant. The inventive GAA composition may be obtained or be obtainable by production in such a plant, especially preferred a bryophyte or moss, especially *Physcomitrella patens.*

The invention also provides a plant or plant cell, especially a bryophythe or bryophyte cell, suitable for producing the inventive GAA. Especially, preferred the plant or cell comprises a transgene encoding the GAA, especially human GAA, suitable for expression in said plant or cell.

The present invention is further described by the following figures and examples, without being limited to these embodiments of the invention.

### Figures

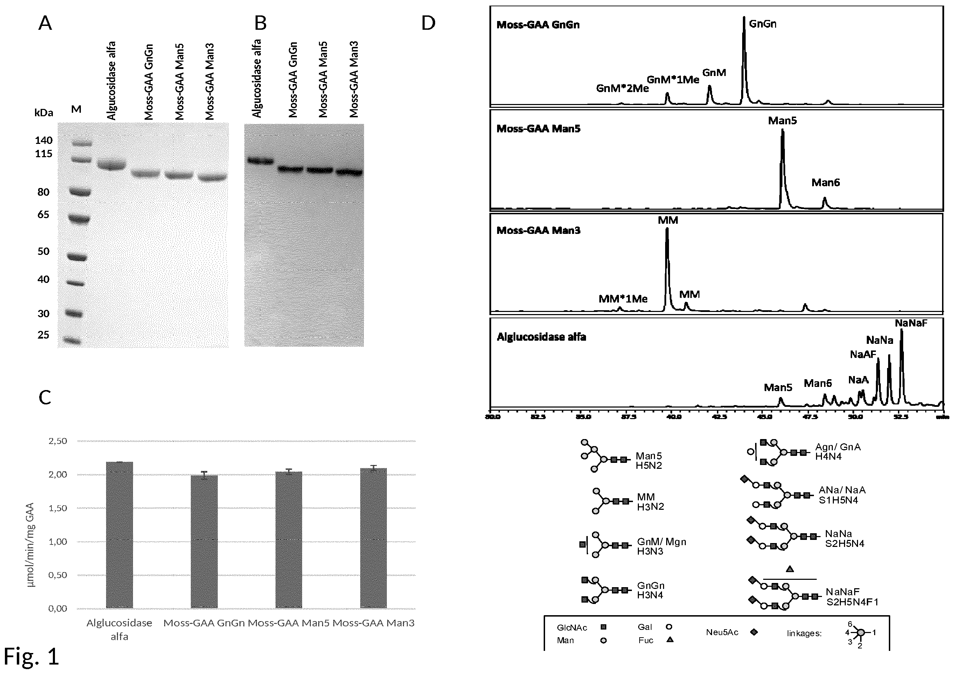

**Figure 1****:** Characterization of moss-GAA variants in comparison to alglucosidase alfa: (A) reducing SDS-PAGE, (B) Western blot using a GAA-specific antibody, (C) specific enzyme activity, (D) total glycan profile via HILIC-HPLC

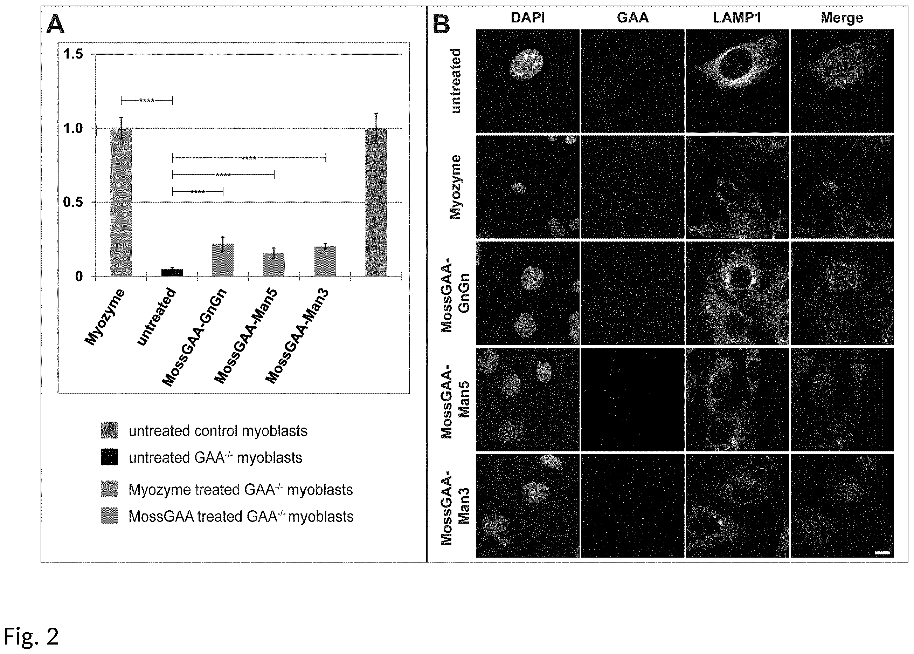

**Figure 2****:** Uptake and activity of rhGAA in immortalized GAA^{-/-}mouse myoblasts. (A) GAA activity assay of GAA^{-/-} mouse myoblasts before and after treatment with all tested rhGAA variants. Data have been normalized to untreated wildtype myoblasts. (B) Immunofluorescence staining of GAA^{-/-} mouse myoblasts for GAA and the lysosomal marker LAMP1 before and after treatment with all tested rhGAA variants. Scale bar 10 µm.

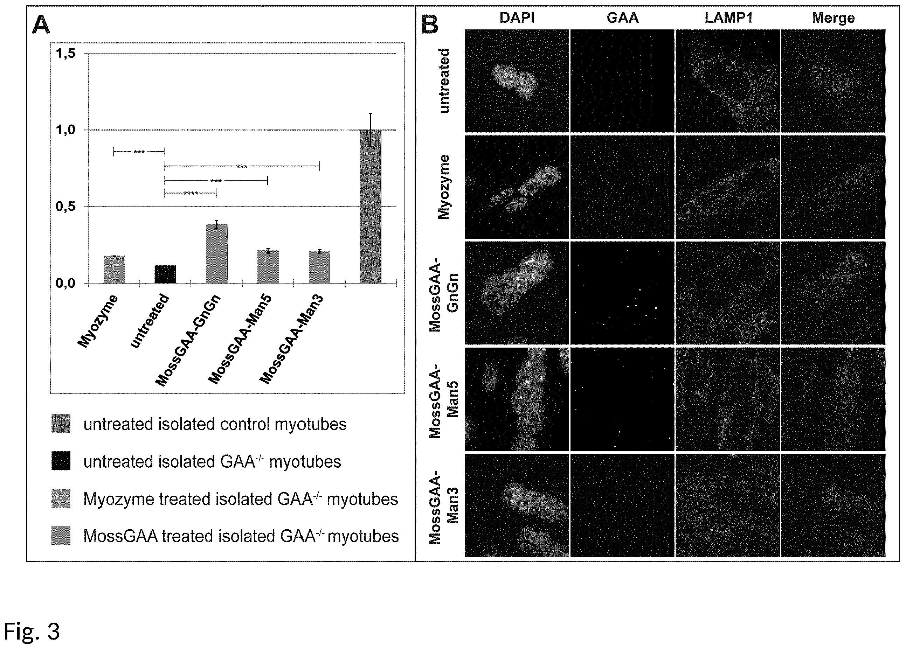

**Figure 3****:** Uptake and activity of rhGAA in immortalized GAA^{-/-}mouse myotubes. (A) GAA activity assay of isolated GAA^{-/-} mouse myotubes before and after treatment with all tested rhGAA variants. Data have been normalized to isolated untreated wildtype myotubes. (B) Immunofluorescence staining of GAA^{-/-} mouse myobtubes for GAA and the lysosomal marker LAMP1 before and after treatment with all tested rhGAA variants. Scale bar 10 µm.

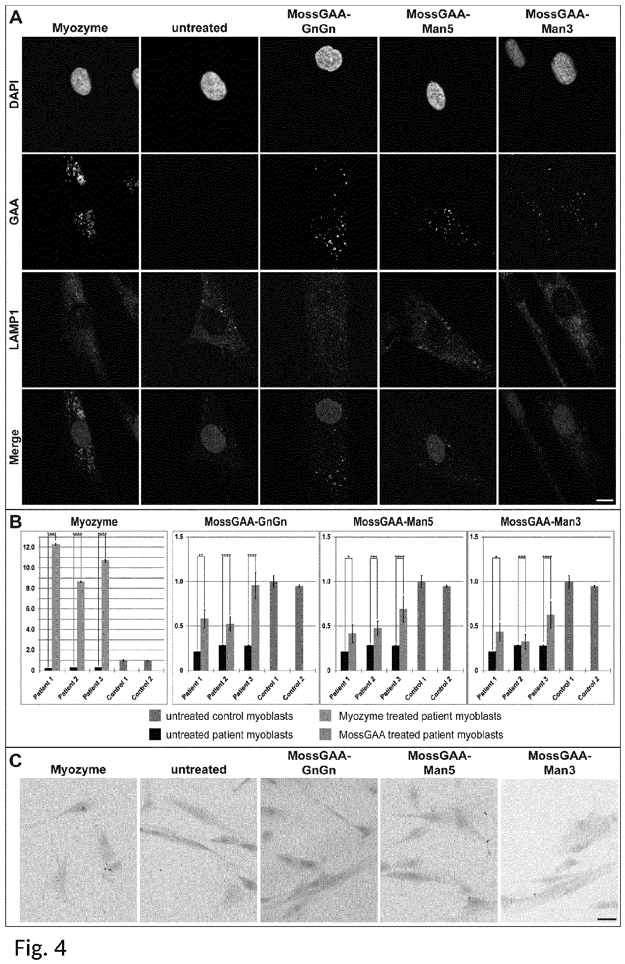

**Figure 4****:** Uptake and activity of rhGAA in human myoblasts. (A) Immunofluorescence staining of Pompe patient primary myoblasts for GAA and the lysosomal marker LAMP1 before and after treatment with all tested rhGAA variants. Scale bar 10 µm. (B) GAA activity assay of Pompe patient primary myoblasts before and after treatment with all tested rhGAA variants. Data have been normalized to untreated control 1. (C) PAS staining of Pompe patient primary myoblasts before and after treatment with all tested rhGAA variants. Scale bar 40 µm.

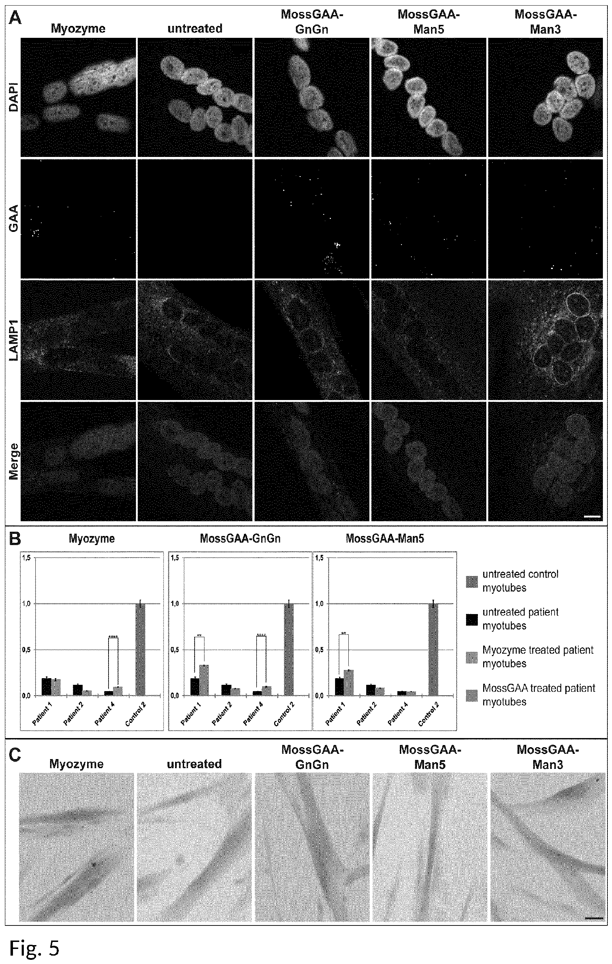

**Figure 5****:** Uptake and activity of rhGAA in human myotubes. (A) Immunofluorescence staining of Pompe patient myotubes for GAA and the lysosomal marker LAMP1 before and after treatment with all tested rhGAA variants. Scale bar 10 µm. (B) GAA activity assay of Pompe patient myotubes before and after treatment with all tested rhGAA variants. Data have been normalized to untreated control 2. (C) PAS staining of Pompe patient myotubes before and after treatment with all tested rhGAA variants. Scale bar 40 µm.

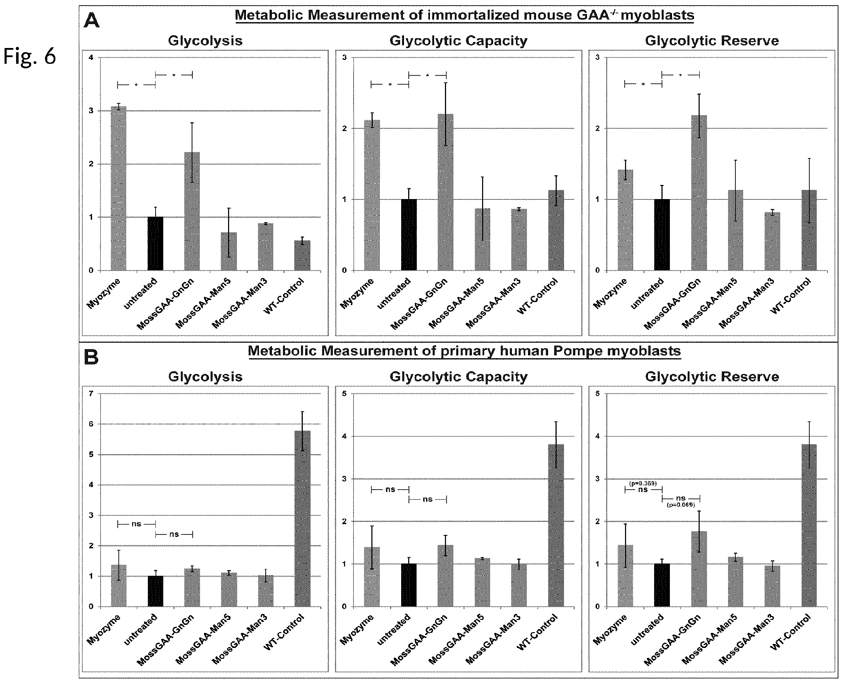

**Figure 6****:** Glycolytic measurements. Measurement of glycolysis, glycolytic capacity and reserve have been performed in (A) immortalized GAA^{-/-} mouse myoblasts (normalized to untreated GAA^{-/-}mouse myoblasts) and a mouse wildtype control; and (B) Pompe patient primary myoblasts (normalized to untreated patient myoblasts) and myoblasts from an unaffected control.

### Examples

### Example 1: Culture of immortalized mouse cells

Immortalized GAA^{-/-} mouse myoblasts cell line LA5 (gained from a GAA-/- mouse, (Raben et al., The Journal of biological chemistry, 1998, 273(30): 19086-92.)). The Myoblasts were cultured in DMEM supplemented with 20% FCS, 10% Horse serum, GlutaMax, 1% Chick embryo extract, IFN-γ and 40 U/ml Penicillin, 0.04 mg/ml Streptomycin in an incubator at 33 °C with 5% CO₂.

Differentiation was started by adding Differentiation medium (DMEM, 2% Horse Serum, GlutaMax, 0.5% Chick embryo extract, 40 U/ml Penicillin and 0.04 mg/ml Streptomycin) to 70% confluent cells at 37 °C with 5% CO₂ over 7 days.

### Example 2: Patients and controls

Primary human myoblasts were obtained from the Muscle Tissue Culture Collection (MTCC) at the Friedrich-Baur-Institute (Department of Neurology, Ludwig-Maximilians-University, Munich, Germany). All control and patient materials were obtained with written informed consent of the donor. Ethical approval was obtained from the ethical review committee at the Ludwig-Maximilians-University, Munich, Germany.

### Example 3: Culture of primary skeletal muscle cells

Primary human myoblasts were cultured in Skeletal Muscle Growth Medium supplemented with SkMC Supplement (PELOBiotech), GlutaMax and 40 U/ml Penicillin, 0.04 mg/ml Streptomycin in an incubator at 37 °C with 5% CO₂. Myoblasts were kept from reaching confluence to avoid differentiation. Passage numbers were matched for controls and patient cells for the respective experiments, throughout all experiments passage numbers 8 to 10 have been used. Coverslips for myoblast immunohistochemistry were fixed at about 60 to 70 % confluence.

For differentiation confluent myoblasts were cultivated for 7 days in DMEM containing 5 % horse serum.

### Example 4: Enzymes

Alglucosidase alfa (Myozyme®, PZN: 04796579) was obtained from Sanofi Genzyme company.

### Example 5: Moss-GAA expression strain construction

DNA sequence of human lysosomal alpha-glucosidase gene (GAA, NCBI Reference: NM_000152.5) starting from bp 511 encoding for a 110 kDa precursor protein was chosen to ensure correct intracellular trafficking to and processing in the lysosomes (Moreland et al., J Biol Chem 2005). The construct was synthesized (Gene-Art, Thermo Fisher Scientific, Regensburg, Germany) in frame with a plant secretory signal peptide sequence and sub-cloned into a moss expression vector. Linear expression cassettes were obtained from the plasmids by restriction. Expression cassettes comprise plant regulatory sequences, plant secretory signal peptide sequence, partial human GAA gene sequence and neomycin-phosphotransferase gene (nptII) under control of cauliflower mosaic virus (CaMV) 35S promoter.

To generate moss cell lines producing the GnGn variant of moss-GAA, protoplasts of a moss double-knockout line devoid of plant specific α-1,3-fucose and β-1,2-xylose residues on its N-glycans (Koprivova et al., Plant Biotechnol J. 2004; 2(6):517-23) were transformed with the expression cassettes by a PEG-based method and were selected using antibiotic geneticin (G418). Drug-resistant moss plantlets were screened for total moss-GAA accumulation per biomass, and the strain with the highest expression was chosen as production strain.

To produce moss-GAA with increased content of terminal mannose (Man5 variant), the above strategy was applied using a recipient strain with additional N-acetylglucosaminyltransferase I gene knock-out.

### Example 6: Production and Purification of moss-GAA

Cultivation of the moss-GAA producing strains (GnGn and Man5) was done as described in (Shen et al., J Inherit Metabol Dis 39:293-303, 2016). The moss was grown in a pure mineral culture medium without any antibiotics or animal-derived component. Because of the lack of C-terminal vacuolar signal, moss-GAA was efficiently secreted into the culture medium. At the end of cultivation, the culture broth was clarified and concentrated. Moss-GAA was purified to homogeneity (>95% via SE-HPLC) using hydrophobic interaction chromatography (Toyopearl Butyl 650-M, Tosoh) followed by ion-exchange chromatography (Toyopearl Sulfate-650F, Tosoh). Subsequently, buffer was exchanged to 25mM Na-Phosphate pH6.2 and moss-GAA was concentrated to approx. 1mg/ml. Purified moss-GAA was stored at -80°C until further use.

### Example 7: Preparation of moss-GAA Man3 variant

To produce the moss-GAA variant with terminal paucomannosidic glycans, the moss-GAA GnGn has been treated with β-N-Acetylglucosaminidase (NEB, P0744, Frankfurt, Germany), following the manufacturers protocol, to specifically cleave off the N-acetylglucosamine motifs.

### Example 8: Analysis of moss-GAA variants

For SDS-Page and western blot, samples were denatured in LDS sample buffer (NuPage, Life Technologies, Carlsbad, USA) at 95°C for 5min (or at 50°C for 5min for western blot) in the presence of dithiothreitol (NuPage reducing agent, Life Technologies). NuPAGE Bis-Tris 4-12% (Life Technologies) were used for protein separation. Western blot was performed using Novex Semi-dry blotter (Life Technologies). Primary antibody used was: rabbit polyclonal antibody to human GAA (Abnova, Taiwan). Secondary antibody: anti-rabbit HRP (abcam, Cambridge, UK).

Specific activity has been measured in an assay using para-nitrophenol-α-D-Glucopyranoside as substrate. Briefly: 50 µl sample was mixed with 450µL reaction buffer, containing 350 µL 25mM Na-Citrat+0.4% BSA, pH 4.0 and 100 µL 20mM para-nitrophenol-α-D-Glucopyranoside (Sigma Aldrich, Germany) and incubated for 60 min at 37°C. Reaction was stopped with 1 mL 200mM boric acid, pH 9.8. Absorbance at 410 nm was read in a 1cm cuvette using SPECORD (Analytik Jena, Jena, Germany) spectrophotometer. Specific enzyme activity was calculated using millimolar extinction coefficient of 18.5 for para-nitrophenol and was expressed as µmol hydrolyzed para-nitrophenol-α-D-Glucopyranoside per min per mg of GAA.

Glycan analysis of moss-GAA variants and alglucosidase alfa was performed using HILIC-HPLC-MS. In short, N-glycans were released from the protein enzymatically using PNGase F. After cleanup and desalting, isolated glycans were labeled using procainamide. Labelled glycans were separated on a Agilent Advance-Bio Glycan Mapping column (2.1 × 150 mm, 1.8 µm) with a Security Guard Ultra precolumn (Phenomenex) on a Nexera X2 HPLC system with a RF-20Axs Fluorescence Detector, equipped with a semimicro flow cell (Shimadzu, Korneuburg, Austria). Solvent A consisted of 80 mM formic acid, buffered to pH 4.4 with ammonia, solvent B of 95% ACN in solvent A. The applied gradient started with an initial hold of solvent B at 99 % for 8 min and a decrease to 57 % B over 60 min, following 25 % B in 2 min, at a flow rate of 0.4 ml min⁻¹; the column oven was set to 45 °C and flow cell thermostat to 45 °C. Fluorescence was measured with wavelengths Ex/Em 310 nm and 370 nm for the procainamide label. 3 µl injection volume was used for HPLC-MS runs with a post-column flow splitter connected to an amaZon ion trap mass spectrometer (Bruker, Bremen, Germany). The ion trap was operated in the positive ion mode with DDA. Target mass was set to m/z 700 and a m/z range from 150 to 1600 in the Enhanced Resolution mode with 150,000 ICC target and 200 ms maximum accumulation time. Data was analyzed in Postrun Analysis with LabSolutions 5.73 (Shimadzu, Germany) and Bruker Daltonics DataAnalysis 4.0.

### Example 9: GAA uptake assay

Myoblasts were grown to 70% confluence, alglucosidase alfa (10µg/ml) / MossGAA variants (10µg/ml each) and 25 mM Na-Phosphat-buffer pH 6.2 (enzyme diluent) as a negative control respectively was added the cells and incubated for 24 h in an incubator at 37 °C with 5% CO₂.

### Example 10: Preparation of cell lysates

After 24 hrs of incubation the medium was aspirated, cells were washed with 1xPBS twice, trypsinized and collected via centrifugation at 1000 g at room temperature. The pellet was resolved in 1ml of 1xPBS and cells were centrifuged for 10 min, at 16100 g, 4°C. PBS was aspirated completely and 100 µl of aqua ad iniectabilia was added. After that ultra-sonification (Sonopuls ultrasonic homogenizer HD2070 with sonotrode MS73, Bandelin) was performed (settings: 30 sec; power 40 sec; 5 cycles). After 10 min centrifugation with 16100 g at 4°C, supernatant was collected in a fresh tube and protein concentration was determined via Nanodrop 1000. Concentration of cell lysate was adjusted to 4µg/µl.

### Example 11: GAA activity assay

For GAA activity assay 20 µl of 4µg/µl cell lysate/standards were used and measured in triplicates on black 96 well plates. To the samples 80 µl of reaction buffer (0.5 mM 4-Methylumbelliferyl alpha-D-Glucopyranoside, 56 mM citric acid, 88mM Na₂HPO₄, 0.4% BSA) was added, mixed for 10 sec at 900rpm and incubated for 60 min at 37°C. Reaction was stopped with stop buffer (0.1 M Glycin, 0.1M NaOH).

Measurement was performed using Tecan Infinite plate reader (orbital mixing step for 5 sec, amplitude 1.5 mm, excitation: 360 nm, emission: 450 nm, gain: 50).

### Example 12: Real time metabolic measurements

Metabolic pathways were assessed using the Seahorse XFp Extracellular Flux Analyzer (Seahorse Bioscience). Myoblasts were seeded in XFp Cell Culture Miniplates (103025-100, Seahorse Bioscience), at a density of 1.5 × 10^4 cells per well and incubated overnight.

To measure glycolytic function, cells were incubated in unbuffered Basal Assay Medium (Seahorse) supplemented with 1 mM glutamine pH 7.4 at 37 °C without CO₂ for 1 h before the assay. Sequential injection of glucose (10 mM), oligomycin (1.0 µ M) and 2-deoxy-D-glucose (50 mM) measures glycolysis, glycolytic capacity, glycolytic reserve and non-glycolytic acidification monitoring Extracellular acidification rate (ECAR). Analysis of the data was performed well-wise.

### Example 13: PAS staining

Cells were fixed with 5% glacial acetic acid in 96% EtOH, rinsed for 1 min in slowly running tap water and immersed in Periodic Acid solution for 5 min at room temperature. Afterwards coverslips were rinsed in several changes of distilled water and immersed in Schiff's Reagent for 15 minutes at room temperature. After washing slides in running tap water for 5 minutes, cells were counterstained in Hematoxylin Solution, for 90 sec and rinsed in running tap water for 5 min. For mounting, fluorescent mounting medium (DAKO) was used.

### Example 14: Immunohistochemistry

Cells were fixed with methanol (-20°C). Primary antibodies used for staining include: GAA (ab166781, abcam) LAMP1 (ab24170, abcam) and secondary antibodies Alexa Fluor 488 donkey antimouse AffiniPure (715-545-151, Jackson ImmunoResearch) and Alexa Fluor 594 goat anti-rabbit (A11012, Invitrogen). DNA was visualized with DAPI (4,6-diamidino-2 phenylindole, dihydrochloride) containing mounting medium (Vector Laboratories, H-1200).

### Example 15: Microscopy and image analysis

All images were obtained using an Olympus FluoView FV1000 / BX 61microscope equipped with a 1.42 NA 60x objective and 3x zoom magnification. Image analysis was performed using ImageJ software.

### Example 16: Statistical Analysis

Statistical significance was determined by two-tailed Student's t test; error bars represent SE. *p ≤ 0.05 was considered statistically significant. ** indicates p values < 0.01; *** indicates p values < 0.001 and **** indicates p values < 0.0001.

### Example 17: Production, purification and characterization of

### moss-GAA variants

The 110kDa human GAA precursor was stably overexpressed in the moss Physcomitrella patens and secreted to the culture medium. The moss-GAA GnGn expressing moss strain was a glycoengineered variant devoid of plant-specific α-1,3-fucose and β-1,2-xylose residues on its N-glycans (Koprivova et al 2004, supra). Two additional glyco-variants of GAA were generated: moss-GAA Man5, expressed in a strain additionally devoid of β-1,2-N-acetylglucosaminyltransferase activity (GnTI knockout) (Shen et al 2015, supra) and moss-GAA Man3, produced by enzymatic removal of N-acetylglucosamine residues from the GnGn variant.

In SDS-PAGE all three glyco-variants of the moss-GAA: GnGn, Man5 and Man3 showed a single major band with a faster mobility than alglucosidase alfa (Fig. 1A), reflecting the lower carbohydrate content in moss-GAA versions (with the lowest in Man3 variant). In western blot all recombinant GAA proteins were detected by a polyclonal anti-human GAA antibody (Fig. 1B). Comparable specific activity could be shown for all three variants and the marketed alglucosidase alfa (Fig. 1C).

HILIC-HPLC-MS analysis revealed a highly homogenous glycosylation profile of all three moss-GAA variants with exclusively mannose or N-acetylglucosamine terminated sugars in sharp contrast to the heterogenous profile of alglucosidase alfa (Fig. 1D and Table 1).

**Table 1:**

| rhGAA | N-glycan structure | % relative abundance | rhGAA | N-glycan structure | % relative abundance |
|---|---|---|---|---|---|
| | | | | | |
| Moss-GAA GnGn | **GnGn** | **63** | Moss-GAA Man3 | **MM** | **76** |
| | GnM | 16 | | AMF | 7 |
| | GnM^{∗}1Me | 8 | | MM^{∗}1Me | 4 |
| | AGnF | 4 | | | 2 |
| | Man6 | 2 | | Man4 | 2 |
| | AGn | 1 | | Man6 | 2 |
| | GnM^{∗}2Me | 1 | | Man5 | 1 |
| | | | | | |
| Moss-GAA Man5 | **Man5** | **77** | | NaNaF | **20** |
| | Man6 | 13 | Alglucosidase alfa | NaNa | 14 |
| | Man5^{∗}1Me | 3 | | NaAF | 12 |
| | Man5^{∗}2Me | 2 | | NaA | 10 |
| | Man7 | <1 | | AA | 5 |
| | | | | AAF | 3 |
| | | | | Man6 | 3 |
| | | | | Man5 | 3 |

### Example 18: Uptake and activity of rhGAA in mouse myoblasts

Immortalized GAA^{-/-} mouse myoblasts were treated with all three moss-GAA variants, alglucosidase alfa, and buffer (untreated) for 24 hrs. After 24 hrs an enzyme activity assay was performed (Fig. 2A). The GAA activity was severely reduced in untreated GAA^{-/-} myoblasts compared to wildtype. Alglucosidase alfa treatment resulted in a highly significant increase of enzyme activity to wildtype levels. All moss-GAA variants showed a highly significant increase in activity over untreated myoblasts as well but reached only about a quarter of the wildtype activity. Moss-GAA GnGn showed the highest activity of the three tested moss-GAA variants.

Immunofluorescence staining for GAA (Fig. 2B) showed faint signals in alglucosidase alfa and moss-GAA GnGn treated cells but little signal in moss-GAA Man5 and Man3.

### Example 19: Uptake and activity of rhGAA in mouse myotubes

As the target organ in the Pompe disease is the mature muscle we tested the uptake of all rhGAA variants in differentiated myotubes. The immortalized GAA^{-/-} mouse myoblasts were differentiated for six days and then treated with all three moss-GAA variants, alglucosidase alfa, and buffer (untreated) for 24 hrs. After 24 hrs myotubes were isolated to avoid contamination of undifferentiated myoblasts, and an enzyme activity assay was performed (Fig. 3A). As expected, the GAA activity was severely reduced in untreated GAA^{-/-} myotubes compared to wildtype. All variants of rhGAA showed a significant increase of GAA activity, but contrary to results in undifferentiated myoblasts none of the variants reached wildtype level. Alglucosidase alfa showed a lower increase of activity compared to moss-GAA; and amongst the tested moss-GAA variants the GnGn type generated the biggest increase in enzyme activity.

Immunofluorescence staining for GAA (Fig. 3B) showed faint signals in moss-GAA GnGn and Man5 treated myotubes but little detectable signal in moss-GAA Man3 and alglucosidase alfa.

### Example 20: Uptake and activity of rhGAA in human myoblasts

Being aware that differences between murine and human cells might affect the outcome of our measurements, we tested the uptake in different primary human myoblasts from Pompe patients in the next step. Primary patient myoblasts were treated with all three moss-GAA variants, alglucosidase alfa, and buffer (untreated) for 24 hrs. After 24 hrs an enzyme activity assay was performed (Fig. 4B). All three patient myoblast cultures showed reduced GAA activity compared to healthy controls - although not to the extent of diseased mouse myoblasts, which can be explained by the remaining enzyme levels in Pompe patients. Alglucosidase alfa showed an 8-13 times increased enzyme activity, to healthy controls levels. All three moss-GAA variants did also show a significant increase in enzyme activity in all three patient myoblast cultures, with the GnGn variant reaching the highest levels, to 50-100% of healthy controls.

Immunofluorescence staining for GAA (Fig. 4A) showed strong signals in alglucosidase alfa and moss-GAA-GnGn treated myoblasts and weak signals in MossGAA-Man5 and -Man3.

Periodic Acid-Schiff (PAS)-staining to detect glycogen depositions (Fig. 4C) showed massive accumulation in untreated cells and a complete clearance of these accumulations following alglucosidase alfa and moss-GAA GnGn treatment. Both moss-GAA Man5 and Man3 variants also led to reduced glycogen accumulations.

### Example 21: Uptake and activity of rhGAA in human myotubes

Next, the uptake of all rhGAA variants in differentiated human patient myotubes was tested. Myoblasts were differentiated for 6 days and then treated with all three moss-GAA variants, alglucosidase alfa, and buffer (untreated) for 24 hrs. After 24 hrs myotubes were isolated to avoid contamination of undifferentiated myoblasts, and an enzyme activity assay was performed (Fig. 5B). The GAA activity was severely reduced in all patient-derived myotubes compared to a healthy control. The enzyme activity increased only in patient 4 cell line following alglucosidase alfa treatment, whereas treatment with moss-GAA Man5 resulted in an increased activity in patient 1 cells and moss-GAA GnGn treatment resulted in an increased activity in patients 1 and 4. We did not test moss-GAA-Man3 due to the amount of differentiated myotubes needed and since we expected it to be similarly or slightly less active as moss-GAA-Man5 in view of the other experiments.

Immunofluorescence staining for GAA in myotubes (Fig. 5A) detected faint signals only following moss-GAA GnGn and Man5 treatment.

PAS-staining in patient-myotubes (Fig. 5C) did show accumulation in untreated cells and only treatment with moss-GAA GnGn led to reduction of these depositions.

### Example 22: Metabolic measurements

To investigate the effect of the recombinant GAA versions on metabolic performance of Pompe cells, we performed measurements of the glycolysis using a Seahorse XFp analyzer (Fig. 6). Due to the limited size of the plate wells, measurements were performed with myoblasts only. Glycolysis in immortalized GAA^{-/-} mouse myoblasts (Fig. 6A) was reduced compared to an immortalized wildtype and treatment with alglucosidase alfa as well as moss-GAA GnGn for 24 hrs resulted in increased glycolysis rate while in case of moss-GAA Man5 and Man3 no significant change could be observed. Following a more detailed data analysis increased glycolytic capacity and reserve for alglucosidase alfa and moss-GAA GnGn treatment could be determined.

To exclude that the immortalization of the mouse myoblasts is affecting the glycolysis of said cells and thus our results, we performed parallel tests with human primary patient myoblasts (Fig. 6B). In this case we could also observe a decreased glycolysis in patient cells compared to healthy controls. Similarly to mouse myoblasts, treatment with alglucosidase alfa and moss-GAA GnGn led to an increased glycolysis rate, which is most evident when looking at the glycolytic reserve. While both treatments resulted in an increase of the glycolytic reserve, measured difference failed to be statistically significant, albeit in case of moss-GAA GnGn only scarcely.

### Example 23: Recapitulation

In the past, evaluation of the cellular uptake of recombinant versions of GAA, as well as other LSD enzymes, has been frequently realized using immortalized patients' fibroblast lines, mainly because they were easily obtained via skin biopsies. It is possible that this focus on fibroblasts, which are abundant of M6P receptors, as cellular models of lysosomal diseases, introduced a certain bias towards the development of M6P-terminated enzymes. Moreover, it is important to point out that fibroblasts do not represent the target cell type of Pompe disease. In recent studies (Basile et al, Journal of Controlled Release 269, 2018: 15-23) use of Pompe myoblasts for cellular uptake tests has been reported. While this is an improvement, myoblasts as progenitor cells can still potentially differ (in e.g.: receptor type and their distribution in the cell membrane) from the fully differentiated myocyte, which is the actual cellular target of the recombinant GAA. Being aware of these shortcomings, we tested the cellular uptake of moss-GAA variants in disease relevant myoblast and differentiated myotubes cell cultures.

We tested the uptake, activity, and effect on glucose metabolism of a non-phosphorylated recombinant human GAA produced in moss (moss-GAA). Three variants of moss-GAA differing in glycosylation pattern have been analyzed: two with terminal mannose residues in a paucimannosidic (Man3) or high-mannose (Man5) configuration and one with terminal N-acetylglucosamine residues (GnGn). Compared to alglucosidase alfa, the moss-GAA GnGn variant showed increased uptake in differentiated myotubes. Moreover, incubation of immortalized muscle cells of Pompe patients with moss-GAA GnGn led to similarly efficient clearance of accumulated glycogen as with alglucosidase alfa. These data suggest that M6P-residues might not always be necessary for the cellular uptake in ERT and show the potential of moss-GAA as more efficient drug for targeting skeletal muscle in Pompe patients.

Testing the uptake and activity of three Moss GAA glyco-variants and comparing them with alglucosidase alfa in immortalized GAA^{-/-} mouse myoblasts and myotubes revealed that in general the uptake into myotubes was less efficient than in myoblasts. While alglucosidase alfa increased enzyme activity to wildtype levels in myoblasts, only very modest activity elevation was observed in myotubes. Of the tested moss-GAAs the GnGn variant performed best in both cell lines and was performing better in myotubes than alglucosidase alfa. Immunofluorescence staining confirmed results of the GAA activity assay in both cell lines, as the detectable signal correlated with the measured activity. This indicates that the enzyme with terminal N-acetlyglucosamines is more efficiently taken up in mouse myotubes than the M6P-terminated one.

As mouse and human cells might basically differ in gene expression and the immortalization process of the mouse cells can have an additional effect, we proceeded to perform the same tests in primary human patient cultures. Overall, we could confirm the results obtained with mouse cells with the only difference being generally higher enzyme activity in myoblasts following treatment, which could indicate either better uptake or lower GAA levels and activity in healthy human myoblasts. The alglucosidase alfa activity reached about ten times the wildtype level and moss-GAA GnGn reached wildtype level in myoblasts. There were however differences between patients suggesting that there are factors affecting the uptake in each individual. This fits well with the observation that alglucosidase alfa treatment has varying affects in Pompe patients. These results were additionally backed up by the PAS-staining, which clearly showed the best reduction of glycogen deposits following the treatment with moss-GAA especially in case of myotubes, where the GnGn variant led to efficient reduction of glycogen accumulations.

It is known, that GAA mutations negatively influence the metabolic functions of the cells. Nevertheless, according to our best knowledge, no study has tackled this subject in detail. We decided to address this question using the Agilent Seahorse technology, providing real-time, quantitative data on metabolic performance of Pompe myoblasts pre- and post- rhGAA treatment. In case of immortalized mouse myoblasts, the GAA knockout showed a surprisingly higher glycolysis rate compared to the wildtype. This might be due to a compensatory effect as all GAA is absent. However, a treatment with both alglucosidase alfa and moss-GAA led to significantly higher glycolysis than in the untreated control and thus was in line with previously acquired results. In human primary myoblasts we measured clearly lower glycolysis in Pompe patients than in healthy controls. In this cell line treatment with alglucosidase alfa and moss-GAA also indicated an improved metabolic performance.

The overall best performance of the GnGn glyco-variant was interesting. In case of a mannose receptor involvement in the endocytosis of moss-GAAs, one would expect a higher uptake rate with mannose-terminated Man3 and Man5 variants than with the GnGn type. Moreover, in an uptake inhibition test with mannan and mannose-6-phosphate, we could observe a clear uptake inhibition of alglucosidase alfa with M6P, but mannan did not lead to full constraint of moss-GAAs internalization. These results suggest a participation of an alternative receptor in the endocytosis of moss-derived GAA.

The present invention demonstrated potential therapeutic effect of moss-derived human GAA, especially in treatment of skeletal muscle pathology in Pompe disease. Moss-GAA GnGn showed superiority over the current standard ERT enzyme: alglucosidase alfa, in cell uptake studies and efficacy in clearance of glycogen accumulations in Pompe patients' myotubes.

## Claims

1. A method of treating a patient with Pompe disease comprising providing a composition of a recombinant acid alpha-glucosidase, which comprises a glycosylation structure of formula 1 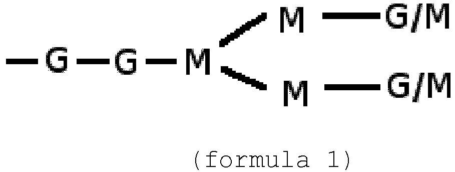
wherein a "G" represents N-acetylglucosamine, a "M" represents mannose, and a "G/M" represents a terminal N-acetylglucosamine or terminal mannose or two terminal mannose subunits, wherein one but not both of the "G/M" subunits may be absent, wherein one or more of the "G" or "M" subunits are not α1,3-fucosylated, not α1,6-fucosylated and not β1,2-xylosylated, and wherein any one of "G", "M" or "G/M" may be methylated; and said glycosylation structure of formula 1 is present in at least 20% of all glycosylation structures of the recombinant acid alpha-glucosidases of the composition; and
administering the composition to the patient.

2. The method of claim 1, wherein the glycosylation structure of formula 1 is glycosylated in at least 10% of the recombinant acid alpha-glucosidase's N-glycosylation sites for the recombinant acid alpha-glucosidases of the composition.

3. The method of claim 1 or 2, wherein at least one G/M of formula 1 is N-acetylglucosamine, preferably wherein both G/M of formula 1 are N-acetylglucosamine.

4. The method of any one of claims 1 to 3, wherein the composition comprises at most 20% of recombinant acid alpha-glucosidase with a glycosylation structure of formula 1 that is at least either α1,3-fucosylated, α1,6-fucosylated or β1,2-xylosylated or a combination thereof.

5. The method of any one of claims 1 to 4, wherein the glycosylation structure of formula 1 is present in at least 30%, preferably at least 40%, even more preferred at least 50%, of all the glycosylation structures of the recombinant acid alpha-glucosidases of the composition.

6. The method of any one of claim 1 to 5, wherein the wherein the composition comprises at least 30% of recombinant acid alpha-glucosidase with a glycosylation structure of formula 1, wherein both G/M are terminal N-acetylglucosamine.

7. The method of any one of claim 1 to 6, wherein the glycosylation structure of formula 1 comprises at most two methylated subunits selected from "G", "M" or "G/M".

8. The method of any one of claims 1 to 7, wherein the glycosylation structure is further defined as a glycosylation structure selected from formulas 2-4: 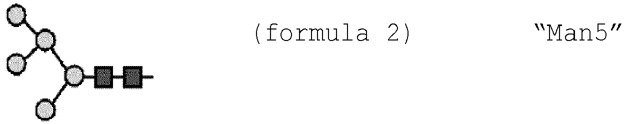 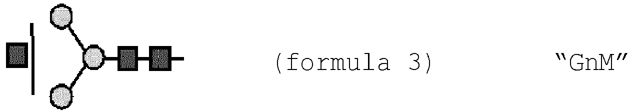 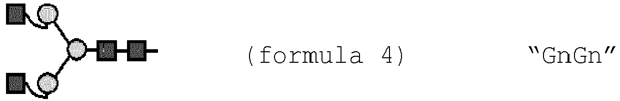 wherein a circle represents mannose and a square represents N-acetylglucosamine, wherein any mannose or N-acetylglucosamine may be methylated.

9. The method of claim 8, wherein the combined amount of the glycosylation structure of formula 3 and formula 4 is at least 70% of all glycosylation structures of the recombinant acid alpha-glucosidases of the composition.

10. The method of claim 8 or 9, wherein the glycosylation structure of formula 4 is present in at least 60% of all glycosylation structures of the recombinant acid alpha-glucosidases of the composition.

11. The method of any one of claims 1 to 10, wherein the patient suffers from muscle loss, in particular skeletal muscle loss and/or heart muscle loss.

12. A composition as defined in any one of claims 1 to 10 for use in the treatment of a patient suffering from Pompe disease, preferably as defined in claim 11.

13. A pharmaceutical composition comprising a recombinant acid alpha-glucosidase, which comprises a glycosylation structure of formula 1 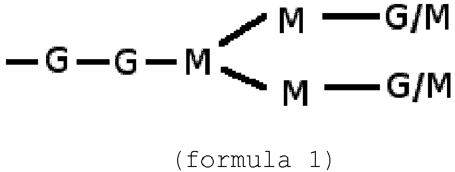 wherein a "G" represents N-acetylglucosamine, a "M" represents mannose, and any one of the "G/M" represents one or two terminal mannose or a terminal N-acetylglucosamine, wherein the other "G/M" is absent or represents a terminal mannose, wherein one or more of the "G" or "M" subunits are not α1,3-fucosylated, not α1,6-fucosylated and not β1,2-xylosylated, and wherein any one of "G", "M" or "G/M" may be methylated; and said glycosylation structure of formula 1 is present in at least 20% of all glycosylation structures of the recombinant acid alpha-glucosidases of the composition.

14. The pharmaceutical composition according to claim 13, wherein the glycosylation structure is further defined as a glycosylation structure selected from formula 2 or formula 3: 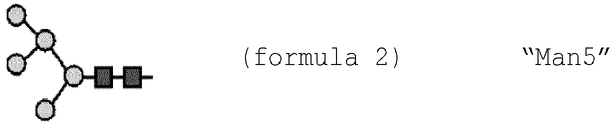 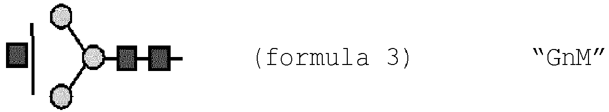 wherein a circle represents mannose and a square represents N-acetylglucosamine, wherein any mannose or N-acetylglucosamine may be methylated, and wherein preferably the glycosylation structure selected from formula 2 is present in at least 60% of all glycosylation structures of the recombinant acid alpha-glucosidases of the composition.

15. The pharmaceutical composition according to claim 13 or 14, wherein the acid alpha-glucosidase bound to a biocompatible polymer, preferably wherein the acid alpha-glucosidase is PEGylated.
